# EUROPEAN PATENT APPLICATION

(11) **EP 4 008 358 A1**
(43) Date of publication of application: **08.06.2022**
(21) Application number: 20860485.0
(22) Date of filing: 01.09.2020
(51) Int. Cl.: A61K 49/22, A61K 41/00, A61K 47/69, A61K 39/395, A61P 35/00, A61K 39/00

(54) **IMMUNE MICROBUBBLE COMPLEX, AND USE THEREOF**

(30) Priority: 02.09.2019 KR 20190108278
(71) Applicant: IMGT Co, Ltd., Seongnam-si, Gyeonggi-do 13605 (KR)
(72) Inventor: KIM, Daehyun, Seongnam-si, Gyeonggi-do 13604 (KR); KIM, Hyun Ryoung, Yongin-si, Gyeonggi-do 17108 (KR); JUNG, Eun Ah, Seoul 06277 (KR)
(74) Representative: Dehns
(86) International application number: PCT/KR2020/011712
(87) International publication number: WO 2021/045485

(57) **Abstract**

The present invention relates to an immune microbubble complex, and a use thereof. An immune-microbubble complex (IMC) according to the present invention comprises microbubbles to which an antibody is conjugated, in which the microbubbles have excellent stability and excellent antibody binding strength, and it was confirmed that, when the immune-microbubble complex is treated with high-intensity focused ultrasound (HIFU), an anti-tumor effect is significantly increased and an immune-enhancing effect is exhibited. Therefore, the immune-microbubble complex according to the present invention is expected to increase the efficiency of delivering the conjugated antibody and be used in both diagnosis and treatment of cancer, and exhibit various functions in the field of immunotherapy, including a contrast effect, half-life improvement, improved drug delivery, a lymphocyte concentration effect, cancer immunotherapy and induction of immunotherapy using ultrasound.

<110>    IMGT CO, LTD.

<120>    Immuno microbubble complex and use thereof

<130>    MPCT20-069

<150>    KR 10-2019-0108278
<151>    2019-09-02

<160>    1

<170>    KoPatentIn 3.0

<210>    1
<211>    290
<212>    PRT
<213>    Artificial Sequence

<220>
<223>    Programmed death ligand-1, PD-L1


<400>    1
Met Arg Ile Phe Ala Gly Ile Ile Phe Thr Ala Cys Cys His Leu Leu
  1               5                  10                  15

Arg Ala Phe Thr Ile Thr Ala Pro Lys Asp Leu Tyr Val Val Glu Tyr
             20                  25                  30

Gly Ser Asn Val Thr Met Glu Cys Arg Phe Pro Val Glu Arg Glu Leu
         35                  40                  45

Asp Leu Leu Ala Leu Val Val Tyr Trp Glu Lys Glu Asp Glu Gln Val
     50                  55                  60

Ile Gln Phe Val Ala Gly Glu Glu Asp Leu Lys Pro Gln His Ser Asn
 65                  70                  75                  80

Phe Arg Gly Arg Ala Ser Leu Pro Lys Asp Gln Leu Leu Lys Gly Asn
                 85                  90                  95

Ala Ala Leu Gln Ile Thr Asp Val Lys Leu Gln Asp Ala Gly Val Tyr
            100                 105                 110

Cys Cys Ile Ile Ser Tyr Gly Gly Ala Asp Tyr Lys Arg Ile Thr Leu
        115                 120                 125

Lys Val Asn Ala Pro Tyr Arg Lys Ile Asn Gln Arg Ile Ser Val Asp
    130                 135                 140

Pro Ala Thr Ser Glu His Glu Leu Ile Cys Gln Ala Glu Gly Tyr Pro
145                 150                 155                 160

Glu Ala Glu Val Ile Trp Thr Asn Ser Asp His Gln Pro Val Ser Gly
                165                 170                 175

Lys Arg Ser Val Thr Thr Ser Arg Thr Glu Gly Met Leu Leu Asn Val
            180                 185                 190

Thr Ser Ser Leu Arg Val Asn Ala Thr Ala Asn Asp Val Phe Tyr Cys
        195                 200                 205

Thr Phe Trp Arg Ser Gln Pro Gly Gln Asn His Thr Ala Glu Leu Ile

210                 215                 220

Ile Pro Glu Leu Pro Ala Thr His Pro Pro Gln Asn Arg Thr His Trp
225                 230                 235                 240

Val Leu Leu Gly Ser Ile Leu Leu Phe Leu Ile Val Val Ser Thr Val
                245                 250                 255

Leu Leu Phe Leu Arg Lys Gln Val Arg Met Leu Asp Val Glu Lys Cys
            260                 265                 270

Gly Val Glu Asp Thr Ser Ser Lys Asn Arg Asn Asp Thr Gln Phe Glu
        275                 280                 285

Glu Thr
    290

## Description

### [Technical Field]

The present invention relates to an immune microbubble complex comprising microbubbles; and an immune checkpoint inhibitory antibody conjugated on the surface of the microbubbles, and a use thereof.

This application claims priority to and the benefit of Korean Patent Application No. 10-2019-0108278, filed on September 02, 2019, the disclosure of which is incorporated herein by reference in its entirety.

### [Background Art]

Cancer is one of the incurable diseases that humans must address, and huge capital is being invested in the development to cure it worldwide, and in Korea, as the No. 1 disease among causes of death caused by diseases, more than 100,000 people are diagnosed and more than 60,000 people die annually.

Cancer treatment methods, which are currently used clinically, include chemotherapy, radiation therapy, targeted therapy and a method of removing a lesion through surgery, and representative examples of chemotherapeutic agents for cancer treatment currently used include doxorubicin, adriamycin, cisplatin, taxol and 5-fluorouracil, which are widely used in chemotherapy for cancer treatment. However, since the methods have limitations and are accompanied by severe side effects and pain to patients rather than a case of complete cure, it is important to develop cancer treatment technology that can minimize the side effects.

Accordingly, cancer immunotherapies have been developed. These methods are methods of killing cancer cells through activation of cancer-specific immune cells, and have a high possibility of a complete cure due to the generation of memory immune cells, can be expected to suppress recurrence, and do not require surgery, so side effects can be minimized compared to conventional cancer treatment methods. The current cancer immunotherapy market is approximately 4 trillion won (KRW), which is expected to surge to 10 trillion won (KRW) by 2024. Up to now, there are approximately 15 cancer immunotherapeutic drugs that have been approved by the FDA being applied in earnest. Among cancer immunotherapeutic methods, representative methods include vaccine treatment and immune checkpoint inhibitor treatment, which are currently known as cancer immunotherapy agents showing good clinical results. However, the currently-used cancer immunotherapeutic methods have a phenomenon in which cancer cells produce their own interfering antibodies and reduce the efficacy of a cancer immunotherapy agents, and therefore, the therapeutic effect is reduced due to the result of losing a certain amount of the administered cancer immunotherapy agents. In addition, the cancer immunotherapy has various side effects and drug loss due to the systemic circulation of drugs.

Meanwhile, theragnostic agents are substances that enable diagnosis and treatment of diseases at the same time. These agents generally consist of small-size materials, and usually has a form in which a fluorescent dye, a radioactive molecule, etc. are supported in a liposome, a polymer or a nanoparticle, and a drug or a diagnostic marker is introduced to the outside. Recently, research on the synthesis of a theragnostic agent consisting of a lipid structure with excellent biocompatibility has been mainly conducted. Particularly, biometric image analysis research using micrometer-size microbubbles (MB; microbubble ultrasound agent) consisting of a lipid structure is being actively conducted, and a variety of research on microbubbles, nanoparticle drug delivery systems and therapeutic methods using a combination therapy thereof has also been made.

Microbubbles exposed to ultrasound cause cavitation which leads to temporary pore formation in the cell membrane of surrounding cells, and because of this, an increase in lymphocytes near cancer cells is shown, and since tumor ablation activates dendritic cells, an effect of activating the immune system through this is shown. Therefore, it was expected that the immunotherapeutic method using ultrasound, as an indirect treatment method, has a narrower range or a lower therapeutic effect than the treatment method using a cancer immunotherapeutic agent when used alone, but can exhibit a synergistic effect when the unique physical mechanism of ultrasound is used in combination with a conventional immunotherapeutic method.

Accordingly, the present inventors have developed a novel immunotherapeutic method by applying a microbubble complex and ultrasound through a combination of ultrasound technology with immunotherapy, which is not yet well known in cancer-related research and development fields, such as cancer diagnosis and treatment.

### [Disclosure]

### [Technical Problem]

The present inventors confirmed that, when antibody-conjugated microbubbles were treated with ultrasound by combining ultrasound technology with a cancer immunotherapeutic method, an excellent antitumor effect is exhibited, compared to when ultrasound is not treated, and based on this, the present invention was completed.

Therefore, the present invention is directed to providing an immune-microbubble complex comprising microbubbles; and an immune checkpoint inhibitory antibody conjugated on the surface of the microbubbles, and a use thereof.

However, technical problems to be solved in the present invention are not limited to the above-described problems, and other problems which are not described herein will be fully understood by those of ordinary skill in the art from the following descriptions.

### [Technical Solution]

To achieve the purpose of the present invention, the present invention provides an immune-microbubble complex comprising microbubbles; and an immune checkpoint inhibitory antibody conjugated on the surface of the microbubbles, which activates T cells by inhibiting the immune evasion of tumor cells in an environment in which an immune response is boosted by ultrasound sonophoresis.

In one embodiment of the present invention, the conjugation between the microbubbles and the antibody may be an amide bond, a bond between thiols, or a biotin-avidin bond.

In another embodiment of the present invention, the microbubbles may comprise 1,2-distearoyl-sn-glycero-3-phosphorylcholine (DSPC) and 1,2-distearoylsn-glycero-3-phosphoethanolamine (DSPE).

In still another embodiment of the present invention, the DSPE may be 1,2-distearoyl-sn-glycero-3-phosphoethanolamine-N-[succinyl(polyethylene glycol)-2000] (DSPE-PEG2000-NHS).

In addition, the present invention provides a method of preparing an immune-microbubble complex, which comprises (a) mixing phospholipids with an organic solvent and then hydrating the resulting mixture;
(b) preparing microbubbles by dispersing and stirring the hydrated liposome precursor obtained in step (a); and
(c) conjugating an immune checkpoint inhibitory antibody to the microbubbles.

In one embodiment of the present invention, the phospholipids may comprise 1,2-distearoyl-sn-glycero-3-phosphorylcholine (DSPC) and 1,2-distearoyl-snglycero-3-phosphoethanolamine (DSPE).

In another embodiment of the present invention, the molar ratio of the DSPC and the DSPE may be 6 to 9:1 to 4 (mol).

In still another embodiment of the present invention, the immune checkpoint inhibitory antibody may be one or more selected from the group consisting of an anti-programmed death ligand-1 (PD-L1) antibody, an anti-B7-1 antibody, and an anti-B7-2 antibody.

In addition, the present invention provides a drug delivery carrier which comprises the immune-microbubble complex.

In addition, the present invention provides a contrast agent composition for cancer cell-specific ultrasound, magnetic resonance imaging or fluorescence analysis, which comprises the immune-microbubble complex.

In addition, the present invention provides a pharmaceutical composition for preventing or treating cancer, which comprises the immune-microbubble complex.

In one embodiment of the present invention, the immune-microbubble complex may be used in combination with ultrasound treatment.

In another embodiment of the present invention, the ultrasound may be high intensity focused ultrasound (HIFU).

In still another embodiment of the present invention, the immune-microbubble complex may be cavitated by ultrasound to concentrate lymphocytes near cancer cells.

In addition, the present invention provides a method of providing information on cancer diagnosis, which comprises treating a biological sample with the immune-microbubble complex; and performing ultrasound treatment.

In addition, the present invention provides a method of diagnosing cancer, which comprises treating a biological sample with the immune-microbubble complex; and performing ultrasound treatment.

In addition, the present invention provides a method of preventing or treating cancer, which comprises administering a composition comprising the immune-microbubble complex to a subject in need; and performing ultrasound treatment.

In addition, the present invention provides a use of a composition comprising the immune-microbubble complex for preventing or treating cancer.

In addition, the present invention provides a use of the immune-microbubble complex for producing a drug used in cancer treatment.

### [Advantageous Effects]

An immune-microbubble complex (IMC) according to the present invention comprises microbubbles to which an antibody is conjugated, in which the microbubbles have excellent stability and excellent antibody binding strength, and it was confirmed that, when the immune-microbubble complex is treated with high-intensity focused ultrasound (HIFU), an anti-tumor effect is significantly increased and an immune-enhancing effect is exhibited. Therefore, the immune-microbubble complex according to the present invention is expected to increase the efficiency of delivering the conjugated antibody and be used in both diagnosis and treatment of cancer, and exhibit various functions in the field of immunotherapy, including a contrast effect, half-life improvement, improved drug delivery, a lymphocyte concentration effect, cancer immunotherapy and induction of immunotherapy using ultrasound.

### [Description of Drawings]

FIG. 1 shows the sizes and numbers of microbubbles (MBs) prepared according to a ratio of DSPC:DSPE-PEG2K-NHS according to one embodiment of the present invention.
FIG. 2 shows the result of observing the stability over time of the microbubbles prepared according to a ratio of DSPC:DSPE-PEG2K-NHS according to one embodiment of the present invention.
FIG. 3 shows the size distribution of the microbubbles prepared according to a ratio of DSPC:DSPE-PEG2K-NHS according to one embodiment of the present invention.
FIG. 4 shows the DSPE-PEG-NHS structure and the size distribution of microbubbles prepared with a DSPC:DSPE-PEG2K-NHS ratio of 9: 1 according to one embodiment of the present invention.
FIG. 5 shows the number of microbubbles according to one embodiment of the present invention depending on time and temperature.
FIG. 6 shows the concentrations of anti-PD-Ll antibody and anti-PD-Ll antibody + micelle in a solution remaining after the synthesis of an immune-microbubble complex (IMC) according to one embodiment of the present invention.
FIG. 7 shows a set of the confocal images of an immune-microbubble complex according to one embodiment of the present invention.
FIG. 8 shows the confocal images and DIC images of an immune-microbubble complex according to one embodiment of the present invention.
FIG. 9 shows the design of the experiment for verifying PD-L1 expression in B16F10 cells according to one embodiment of the present invention.
FIG. 10 shows the result of verifying PD-L1 expression in B16F10 cells according to one embodiment of the present invention.
FIG. 11 shows an anti-tumor effect by high-intensity focused ultrasound (HIFU) treatment on the microbubbles and immune-microbubble complex according to one embodiment of the present invention.
FIG. 12 shows IFN-γ and cytolytic T cell activity according to HIFU treatment of the microbubbles and immune-microbubble complex according to one embodiment of the present invention.

### [Modes of the Invention]

The present invention provides an immune-microbubble complex, comprising microbubbles; and an immune checkpoint inhibitory antibody conjugated on the surface of the microbubbles, which activates T cells by suppressing immune evasion of tumor cells in an environment in which an immune response is boosted by ultrasound sonophoresis.

According to the present invention, the microbubbles may boost an immune response by ultrasound sonophoresis, and the immune checkpoint inhibitory antibody may allow immune cells to recognize target disease cells.

In the present invention, the microbubbles may comprise 1,2-distearoyl-snglycero-3-phosphorylcholine (DSPC) and 1,2-distearoyl-sn-glycero-3-phosphoethanolamine (DSPE).

In the present invention, the antibody may be conjugated to microbubbles by a linker or an activated functional group on the surface of microbubbles. The functional group may be a thiol group or an amine group, and the linker may be a compound comprising the functional group, and the conjugation of an antibody with the microbubbles may be an amide bond, a bond between thiols, or a biotin-avidin bond.

In the present invention, the "linker" refers to a material that connects an antibody or drug to microbubbles, and may be, but not limited to, for example, N-hydroxysuccinimide (NHS) or maleimide. According to one embodiment of the present invention, the DSPE may be 1,2-distearoyl-sn-glycero-3-phosphoethanolamine-N-[succinyl(polyethylene glycol)-2000] (DSPE-PEG2000-NHS) to which NHS is connected as a linker .

In the present invention, the particle diameter of the microbubble may be 0.8 to 1.5 µm, and according to one embodiment of the present invention, the average diameter of the microbubbles may be 1.19±0.245 µm.

In the present invention, a molar ratio of DSPC and DSPE may be 6 to 9:1 to 4 (mol), 6.5 to 9:1 to 3.5 (mol), or 7 to 9:1 to 3 (mol), and preferably, 7:3 (mol) or 9:1 (mol). Most preferably, when microbubbles are prepared in a molar ratio of 9:1 (mol), the stability of the prepared microbubbles may be the highest, but the present invention is not limited thereto.

The term "ultrasound" used herein refers to a sound wave that exceeds a frequency of 16 Hz to 20 kHz, which is generally audible to the ears of a human, and high intensity focused ultrasound (HIFU) may exhibit an instantaneous thermal effect (65 to 100 °C), a cavitation effect, a mechanical effect and a sonochemical effect according to energy and a frequency by introducing focused ultrasound providing continuous, high-intensity ultrasound energy to the focal point. Ultrasound is harmless when passing through human tissues, but high-intensity ultrasound that forms the focal point generates sufficient energy to cause coagulation necrosis and thermal cauterization regardless of the type of tissue.

In the present invention, the ultrasound may be HIFU, but the present invention is not limited thereto. In the present invention, the frequency used for HIFU may be 0.1 to 10 Mhz, 0.1 to 9 Mhz, 0.1 to 8 Mhz, 0.1 to 7 Mhz, 0.1 to 6 Mhz, 0.1 to 5 Mhz, 0.1 to 4 Mhz, 0.1 to 3 Mhz, or 0.1 to 2 Mhz, and according to one embodiment of the present invention, 1.1 Mhz. In the present invention, the HIFU may be treated for 10 to 20 seconds under conditions of 1.1 Mhz, 50 to 100 W, 1 to 10 duty cycles, and 40 PRF, but the present invention is not limited thereto.

The term "sonophoresis" used herein uses sound waves, usually, wave energy of ultrasound to improve the transport of materials passing through a liquid medium, and refers to a drug delivery method using ultrasound to increase drug absorption. Here, compression waves of sound waves cause "flow" and/or "cavitation" in a liquid medium. When the immune checkpoint inhibitory antibody according to the present invention is treated with ultrasound, an immune response boosting effect is exhibited by sonophoresis, and the antibody may be further targeted to target cells such as cancer cells.

The term "cavitation" used herein refers to a phenomenon in which, when there is a low pressure area in a fluid, a gas included in water escapes from water and is concentrated in the low-pressure area, resulting in an empty space without water, and as ultrasound-induced cavitation occurs in the immune-microbubble complex in the present invention, voids are temporarily formed in the cell membrane of surrounding cells, and materials are permeated into the cells through the voids, thereby concentrating lymphocytes near cancer cells.

In addition, the present invention provides a method of preparing an immune-microbubble complex, which comprises (a) mixing phospholipids with an organic solvent and then hydrating the resulting mixture;
(b) preparing microbubbles by dispersing and stirring the hydrated liposome precursor obtained in step (a); and
(c) conjugating an immune checkpoint inhibitory antibody to the microbubbles.

In the present invention, the phospholipid may comprise DSPC and DSPE.

In the present invention, the molar ratio of DSPC and DSPE in the phospholipid may be 6 to 9:1 to 4 (mol), 6.5 to 9:1 to 3.5 (mol), or 7 to 9:1 to 3 (mol), preferably 7:3 (mol) or 9:1, and most preferably, 9:1 (mol).

The term "checkpoint" is a protein used in the procedure of activating or inactivating immune cells of the human body, comprising, for example, programmed death ligand-1 (PD-L1) located on the surface of cancer cells, B7-1 and B7-2. In the present invention, the "checkpoint inhibitory" means the capacity of inhibiting an immune system inhibitory checkpoint, and the blockage of an inhibited immune checkpoint activates an immune system function. In the present invention, the antibody conjugated to microbubbles may serve as a checkpoint inhibitor.

The term "antibody" used herein refers to a polypeptide including a framework region derived from an immunoglobulin gene recognized by specifically binding to an antigen or a fragment thereof. The recognized immunoglobulin gene includes genes of kappa (κ), lambda (λ), alpha (α), gamma (γ), delta (δ), epsilon (ε) and mu (µ) constant domains as well as numerous genes of immunoglobulin variable domains. Light chains are classified into κ or λ. Heavy chains are classified into γ, µ, α, δ, and ε, designating the classes of immunoglobulins, such as IgG, IgM, IgA, IgD and IgE. Typically, the antigen-binding region of an antibody is most critical for binding specificity and affinity. In the present invention, antibodies or fragments thereof may be derived from different subjects, including a human, a mouse, a rat, a hamster, a camel, and a rabbit, but the present invention is not limited thereto. The antibody of the present invention may include an antibody modified or mutated at one or more amino acid positions to improve or adjust a preferable function of the antibody (e.g., glycosylation, expression, antigen recognition, effector function, antigen binding or specificity).

In the present invention, the antibody may be one or more selected from the group consisting of an anti-PD-Ll antibody, an anti-B7-1 antibody, and an anti-B7-2 antibody, and according to one embodiment of the present invention, the antibody may be an anti-PD-Ll antibody, but the present invention is not limited thereto.

In the present invention, the anti-PD-Ll antibody may specifically bind to PD-L1, thereby inhibiting PD-L1 binding and preventing the inhibition of an immune response to a tumor. Here, the amino sequence of PD-L1 may be represented by SEQ ID NO: 1 (NCBI GenBank: ADK70950.1), and as the anti-PD-Ll antibody, InVivoPlus anti-mouse PD-L1(B7-H1)(Catalog# BP0101, Clone 10F.9G2) was purchased.

In addition, the present invention provides a drug delivery carrier comprising the immune-microbubble complex.

The term "drug" used herein refers to any compound having desired biological activity. The desired biological activity includes any activity useful for the diagnosis, cure, alleviation, treatment or prevention of a disease in a human or other animals.

In addition, the present invention provides a contrast agent composition for cancer cell-specific ultrasound, magnetic resonance imaging or fluorescence analysis, which comprises the immune-microbubble complex.

The term "contrast agent" used herein refers to a material administered to a body to potently and specifically contrast or image cancer cells *in vivo*, and is now widely used for image enhancement of tissues and cells in medical and diagnostic fields. The term "contrast agent" used herein is not limited to the range of conventionally known CT, PET and MRI contrast agents, and includes contrast agents for ultrasound imaging and fluorescent imaging.

In addition, the present invention provides a pharmaceutical composition for preventing or treating cancer, which comprises the immune-microbubble complex.

The term "cancer" used herein is the generic term for diseases caused by cells having an aggressive property in which cells divide and grow without respect to the limits of normal growth, an invasive property in which cells penetrate into surrounding tissue, and a metastatic property in which cells spread to other parts of the body.

In the present invention, the cancer is not particularly limited as long it is known as a malignant tumor in the art, and may be selected from the group consisting of breast cancer, colorectal cancer, lung cancer, small cell lung cancer, stomach cancer, liver cancer, blood cancer, bone cancer, pancreatic cancer, skin cancer, head or neck cancer, skin or intraocular melanoma, uterine cancer, ovarian cancer, rectal cancer, anal cancer, colon cancer, fallopian tube carcinoma, endometrial carcinoma, cervical cancer, vaginal cancer, vulvar carcinoma, Hodgkin's disease, esophageal cancer, small intestine cancer, endocrine adenocarcinoma, thyroid cancer, parathyroid cancer, adrenal cancer, soft tissue sarcoma, urethral cancer, penile cancer, prostate cancer, chronic or acute leukemia, lymphocyte lymphoma, bladder cancer, kidney or ureter cancer, renal cell carcinoma, renal pelvic carcinoma, a CNS tumor, primary CNS lymphoma, a spinal cord tumor, brainstem glioma and pituitary adenoma.

In the present invention, the "prevention" refers to all actions of inhibiting cancer or delaying the onset thereof by administration of the composition according to the present invention.

The "treatment" used herein refers to all actions involved in alleviating or beneficially changing symptoms of cancer by administration of the composition according to the present invention.

The term "pharmaceutical composition" used herein refers to one prepared for preventing or treating cancer, and may further include a suitable carrier, excipient and diluent, which are conventionally used in preparation of a pharmaceutical composition. The excipient may be one or more selected from the group consisting of a diluent, a binder, a disintegrant, a lubricant, an adsorbent, a humectant, a film-coating material, and a controlled-release additive.

The pharmaceutical composition according to the present invention may be formulated in the form of a powder, a granule, a sustained-release granule, an enteric granule, a solution and a liquid, an ophthalmic solution, an elixir, an emulsion, a suspension, a spirit, a troche, aromatic water, lemonade, a tablet, a sustained-release tablet, an enteric tablet, a sublingual tablet, a hard capsule, a soft capsule, a sustained-release capsule, an enteric capsule, a pill, a tincture, a soft extract, a dry extract, a fluid extract, an injection, a capsule, a perfusate, a plaster, a lotion, a paste, a spray, an inhalant, a patch, a sterile injection, or an external preparation such as an aerosol according to a conventional method, and the external preparation may have a formulation such as a cream, a gel, a patch, a spray, an ointment, a plaster, a lotion, a liniment, a paste or a cataplasma.

As a carrier, an excipient and a diluent that may be included in the pharmaceutical composition according to the present invention, lactose, dextrose, sucrose, oligosaccharide, sorbitol, mannitol, xylitol, erythritol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate and mineral oil may be used.

The composition may be formulated with a diluent or an excipient such as a filler, a thickening agent, a binder, a wetting agent, a disintegrant, a surfactant, which are conventionally used.

As additives for a tablet, powder, granule, capsule, pill and troche, excipients such as corn starch, potato starch, wheat starch, lactose, sucrose, glucose, fructose, dimannitol, precipitated calcium carbonate, synthetic aluminum silicate, calcium monohydrogen phosphate, calcium sulfate, sodium chloride, sodium bicarbonate, purified lanolin, microcrystalline cellulose, dextrin, sodium alginate, methylcellulose, carboxymethylcellulose, sodium carboxymethylcellulose, kaolin, urea, colloidal silica gel, hydroxypropyl starch, hydroxypropyl methyl cellulose, 1928, 2208, 2906, 2910, propylene glycol, casein, calcium lactate and Primojel; binders such as gelatin, gum arabic, ethanol, agar powder, cellulose acetate phthalate, carboxymethyl cellulose, carboxymethyl cellulose calcium, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch powder, hydroxypropylcellulose, hydroxypropylmethylcellulose, polyvinyl alcohol and polyvinylpyrrolidone; disintegrants such as hydroxypropylmethylcellulose, corn starch, agar powder, methylcellulose, bentonite, hydroxypropyl starch, sodium carboxymethylcellulose, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, sucrose, magnesium aluminum silicate, a di-sorbitol solution and light anhydrous silicic acid; and lubricants such as calcium stearate, magnesium stearate, stearic acid, hydrogenated vegetable oil, talc, limestone kaolin, petrolatum, sodium stearate, cacao butter, sodium salicylate, magnesium salicylate, polyethylene glycol 4000 and, 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, a higher fatty acid, a higher alcohol, silicone oil, paraffin oil, polyethylene glycol fatty acid ether, starch, sodium chloride, sodium acetate, sodium oleate, dileucine and light anhydrous silicic acid may be used.

Additives for a liquid according to the present invention may be water, diluted hydrochloric acid, diluted sulfuric acid, sodium citrate, monostearate sucrose, polyoxyethylene sorbitol fatty acid esters (Tween esters), polyoxyethylene monoalkylethers, lanolin ethers, lanolin esters, acetic acid, hydrochloric acid, acetic acid, hydrochloric acid, aqueous ammonia, ammonium carbonate, potassium hydroxide, sodium hydroxide, prolamine, polyvinylpyrrolidone, ethyl cellulose, and sodium carboxymethylcellulose.

For a syrup according to the present invention, a sucrose solution, other type of sugars or sweeteners may be used, and a flavoring agent, a coloring agent, a preservative, a stabilizer, a suspending agent, an emulsifier or a thickener may be used as needed.

For an emulsion according to the present invention, an emulsifier, a preservative, a stabilizer, or a flavoring agent may be used as needed.

For a suspension according to the present invention, a suspending agent such as acacia, tragacanth, methylcellulose, carboxymethylcellulose, sodium carboxymethylcellulose, microcrystalline cellulose, sodium alginate, hydroxypropylmethylcellulose, 1828, 2906 or 2910 may be used, and a surfactant, a preservative, a stabilizer, a coloring agent, a flavoring agent may be used as needed.

For an injection according to the present invention, a solvent such as injectable sterile water, 0.9% sodium chloride for injection, Ringer's solution, a dextrose for injection, dextrose+sodium chloride for injection, PEG, lactated Ringer's solution, ethanol, propylene glycol, non-volatile oil-sesame oil, cottonseed oil, peanut oil, soybean oil, corn oil, ethyl oleate, isopropyl myristic acid or benzene benzoate; a solubilizing agent such as sodium benzoate, sodium salicylate, sodium acetate, urea, urethane, monoethylacetamine, butazolidine, propylene glycol, Tween, nicotinamide, hexamine or dimethylacetamide; a buffer such as a weak acid and a salt thereof (acetic acid and sodium acetate), a weak base and a salt thereof (ammonia and ammonium acetate), an organic compound, a protein, albumin, peptone, or gums; an isotonic agent such as sodium chloride; a stabilizer such as sodium bisulfite (NaHSO₃), carbon dioxide gas, sodium metabisulfite (Na₂S₂O₃), sodium sulfite (Na₂SO₃), nitrogen gas (N₂) or ethylenediaminetetracetic acid; an antioxidant such as sodium bisulfide 0.1%, sodium formaldehyde sulfoxylate, thiourea, disodium ethylenediaminetetraacetate or acetone sodium bisulfite; a pain-relief agent such as benzyl alcohol, chlorobutanol, procaine hydrochloride, glucose or calcium gluconate; or a suspending agent such as sodium CMC, sodium alginate, Tween 80 or aluminum monostearate, may be used.

For a suppository according to the present invention, a base such as cacao butter, lanolin, Witepsol, polyethylene glycol, glycerogelatin, methyl cellulose, carboxymethylcellulose, a mixture of stearate and oleate, Subanal, cottonseed oil, peanut oil, palm oil, cacao butter + cholesterol, lecithin, Lanette wax, glycerol monostearate, Tween or Span, Imhausen, monolene (propylene glycol monostearate), glycerin, Adeps solidus, Buytyrum Tego-G, Cebes Pharma 16, hexalide base 95, Cotomar, Hydrokote SP, S-70-XXA, S-70-XX75 (S-70-XX95), Hydrokote 25, Hydrokote 711, Idropostal, Massa estrarium, A, AS, B, C, D, E, I, T), Mass-MF, Masupol, Masupol-15, neosuppostal-N, paramount-B, supposiro (OSI, OSIX, A, B, C, D, H, L), suppository base IV types (AB, B, A, BC, BBG, E, BGF, C, D, 299), Suppostal (N, Es), Wecoby (W, R, S, M ,Fs), or Tegester triglyceride base (TG-95, MA, 57) may be used.

A solid formulation for oral administration may be a tablet, pill, powder, granule or capsule, and such a solid formulation may be prepared by mixing at least one of excipients, for example, starch, calcium carbonate, sucrose, lactose and gelatin, with the active ingredient. Also, in addition to the simple excipient, lubricants such as magnesium stearate and talc may also be used.

As a liquid formulation for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative may be included. A formulation for parenteral administration may be a sterilized aqueous solution, a non-aqueous solvent, a suspension, an emulsion, a lyophilizing agent or a suppository. As the non-aqueous solvent or suspension, propylene glycol, polyethylene glycol, a vegetable oil such as olive oil, or an injectable ester such as ethyl oleate may be used.

The pharmaceutical composition of the present invention is administered at a pharmaceutically effective amount. The "pharmaceutically effective amount" used herein refers to an amount sufficient for treating a disease at a reasonable benefit/risk ratio applicable for medical treatment, and an effective dosage may be determined by parameters including a type of a patient's disease, severity, drug activity, sensitivity to a drug, administration time, an administration route and an excretion rate, the duration of treatment and drugs simultaneously used, and other parameters well known in the medical field.

The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without a side effect, and such a dose may be easily determined by one of ordinary skill in the art.

The pharmaceutical composition of the present invention may be administered to a subject in need via various routes. All administration routes may be expected, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention is determined according to a variety of related parameters, including a disease to be treated, an administration route, a patient's age, sex and weight, and the severity of a disease, and the type of a drug as an active ingredient.

In the pharmaceutical composition for preventing or treating cancer according to the present invention, the immune-microbubble complex comprised in the composition may be used in combination with ultrasound treatment, and the composition may further comprise one or more selected from the group consisting of an anticancer agent, an imaging contrast agent, an antibiotic, an anti-inflammatory agent, a protein, a cytokine, a peptide and an antibody, other than the immune-microbubble complex.

In addition, the present invention provides a method of providing information on cancer diagnosis, which comprises treating a biological sample with an immune-microbubble complex; and performing ultrasound treatment.

In addition, the present invention provides a method of diagnosing cancer, which comprises treating a biological sample with an immune-microbubble complex; and performing ultrasound treatment.

The term "diagnosis" used herein refers to identification of the presence or characteristics of a pathological condition. For the purpose of the present invention, diagnosis is to confirm whether cancer has developed.

In addition, the present invention provides a method of treating cancer, which comprises administering a composition comprising the immune-microbubble complex to a subject in need; and performing ultrasound treatment.

In addition, the present invention provides a use of a composition comprising the immune-microbubble complex for treating cancer.

In addition, the present invention provides a use of the immune-microbubble complex for producing a drug, which is used in cancer treatment.

In the present invention, the "administration" refers to providing the composition of the present invention to a subject in need by any suitable method.

In the present invention, the term "subject" used herein refers to a target in need of treatment, and more specifically, a mammal such as a human or a non-human primate, a mouse, a dog, a cat, a horse, or a cow.

In one embodiment of the present invention, microbubbles were prepared with various ratios of 1,2-disteaoyl-sn-glycero-3-phosphocholine (DSPC) and 1,2-distearoyl-sn-glycero-3-phosphoethanolamine-N-[succinyl(polyethylene glycol)-2000](DSPE-PEG2K-NHS), and the physical properties of the microbubbles were compared. It was confirmed that when microbubbles are prepared with DSPC and DSPE-PEG2K-NHS in a molar ratio of 9:1, the microbubbles have the highest stability (refer to Example 2).

Tumor cells evade an immune response using their physiological method, and on the surface of the tumor cells, PD-L1 is normally upregulated, and on the surface of T cells, programmed death 1 (PD-1) induces an immune response to PD-L1/PD-1 binding and inhibition of the involvement of a co-stimulatory molecule CD80. CD80 is a member of the immunoglobulin superfamily that provides an antigen-non-specific co-stimulatory signal important for a maximum immune response. When such a receptor binds with a ligand, Src homology 2 domain-containing protein tyrosine family phosphatases (SHPs) are recruited, which renders T cells incapable of releasing granules and perforins regardless of the recognition of MHC I, and inhibits regulatory T cell stimulation, the promotion of T cell apoptosis, and effector T cell activation.

The inhibition of PD-L1/PD-1 binding is one of the methods capable of maintaining an anti-tumor immune response by suppressing immune reduction, and an immunotherapy targeting PD-L1/PD-1 binding may be used.

Therefore, in another embodiment of the present invention, an immune-microbubble complex (IMC) was prepared by binding an anti-PD-Ll antibody to the prepared microbubbles, and it was confirmed that, by measuring the conjugation efficiency of the anti-PD-Ll antibody, the antibody is 100% conjugated (refer to Example 3).

In still another embodiment of the present invention, by observing a confocal image of the prepared immune-microbubble complex (IMC), an antibody conjugated to the surface of microbubbles was identified (refer to Example 4).

In yet another embodiment of the present invention, as a result of confirming an anti-tumor effect and the activity of IFN-γ and cytolytic T cells, shown by treating the prepared immune-microbubble complex (IMC) with HIFU, it was confirmed that, when the IMC is treated with HIFU, an immune-enhancing effect is shown (refer to Example 5).

Hereinafter, to help in understanding the present invention, exemplary examples will be suggested. However, the following examples are merely provided to more easily understand the present invention, and not to limit the present invention.

### Example 1. Preparation of materials

1,2-disteaoyl-sn-glycero-3-phosphocholine (DSPC) was obtained from Coatsome, and 1,2-distearoyl-sn-glycero-3-phosphoethanolamine-N-[succinyl(polyethylene glycol)-2000] (DSPE-PEG2000-NHS) was purchased from Nanocs. In addition, a rat IgG2b isotype control (7.62 mg/ml) and anti-mouse PD-L1(B7-H4)(6.76 mg/ml) were obtained from BioXcell.

### Example 2. Preparation of microbubbles and confirmation of properties of microbubbles according to DSPE-PEG2K ratio

### 2-1. Preparation of microbubbles

According to a method of hydrating a phospholipid film using 1,2-disteaoylsn-glycero-3-phosphocholine (DSPC) and 1,2-distearoyl-sn-glycero-3-phosphoethanolamine-N-[succinyl(polyethylene glycol)-2000] (DSPE-PEG2K-NHS), microbubbles (hereinafter, MBs) were synthesized, and at this time, the properties of the microbubbles prepared according to ratios of DSPC and DSPE-PEG2K-NHS were compared.

Therefore, DSPC and DSPE-PEG2K-NHS were dissolved in chloroform in a molar ratio of 9:1, 7:3, 6:4, 5:5, or 3:7. Subsequently, the chloroform was evaporated, thereby forming a phospholipid film. 1.0 mg/ml of the phospholipid film was disrupted using a bath sonicator, and hydrated with 0.01M PBS at a phase transition temperature of DSPC. Afterward, the hydrated result was put into a vial, and filled with sulfur hexafluoride gas (SF₆). Subsequently, to transform the hydrated liposome precursor into microbubbles, the hydrated liposome precursor was stirred using Vialmix^{™} (Definity, USA) for 45 seconds, thereby preparing microbubbles.

The size distribution and zeta potential of the microbubbles according to a ratio of DSPC:DSPE-PEG2K-NHS prepared by the above-described method were measured using Malvern Zetasizer Nano (Malvern Instrument Ltd., Worcesterchire, U.K.), the microbubbles were dissolved in PBS (pH 7.4) at 100 µg/ml, and the measurement was performed five times at 25 °C.

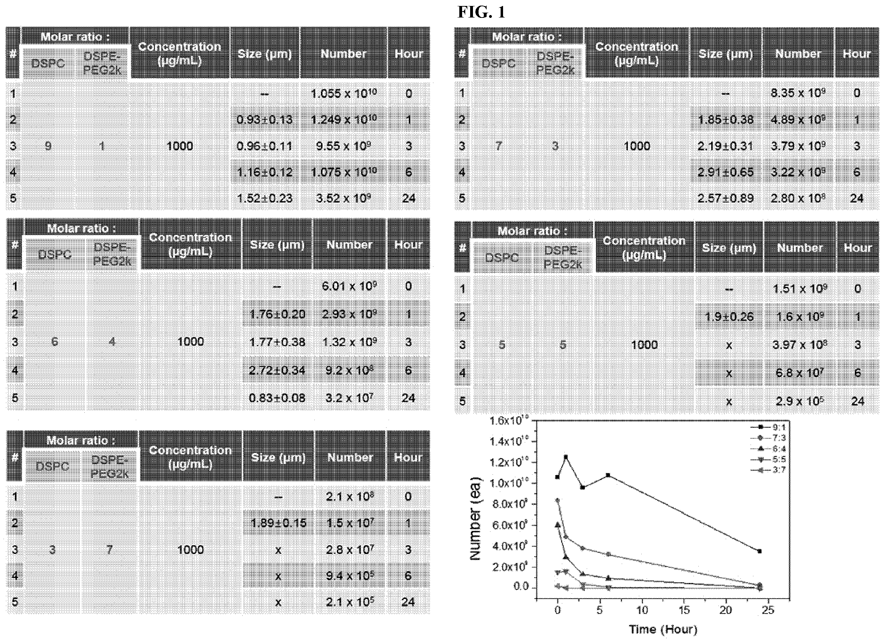

As a result, the sizes and numbers of the microbubbles prepared in five different ratios are shown in FIG. 1, and it was confirmed that when the molar ratio of DSPC:DSPE-PEG2K-NHS is 9:1, smaller and a higher number of microbubbles are generated.

### 2-2. Comparison of microbubble stability

The stability of microbubbles prepared according to the ratios of DSPC:DSPE-PEG2K-NHS in Example 2-1 was observed over time.

Specifically, microbubbles were prepared in five different molar ratios as shown in Example 2-1, and the degree of disappearance of the microbubbles was visually confirmed at room temperature for 0, 3, 6, and 24 hours.

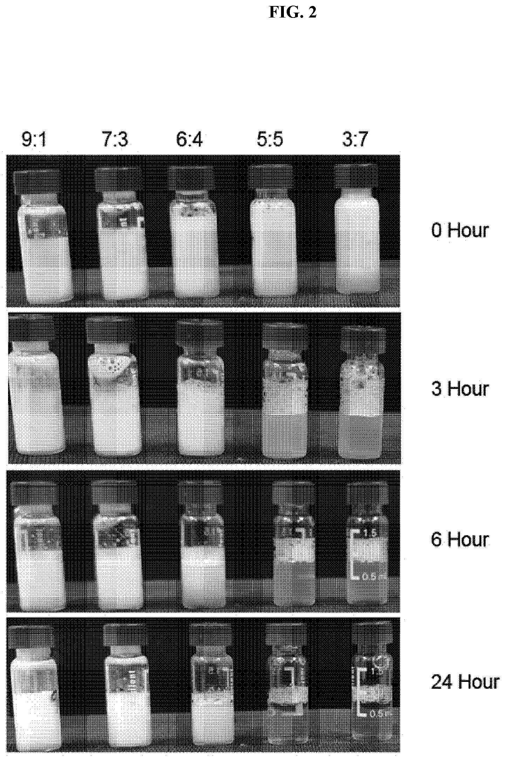

As a result, as shown in FIG. 2, with the lapse of time, by observing that the microbubbles disappeared as the solutions became transparent in the order of the 3:7, 5:5 and 6:4 ratios of DSPC:DSPE-PEG2K-NHS, it was confirmed that the microbubbles are stable when prepared in ratios of 7:3 and 9:1.

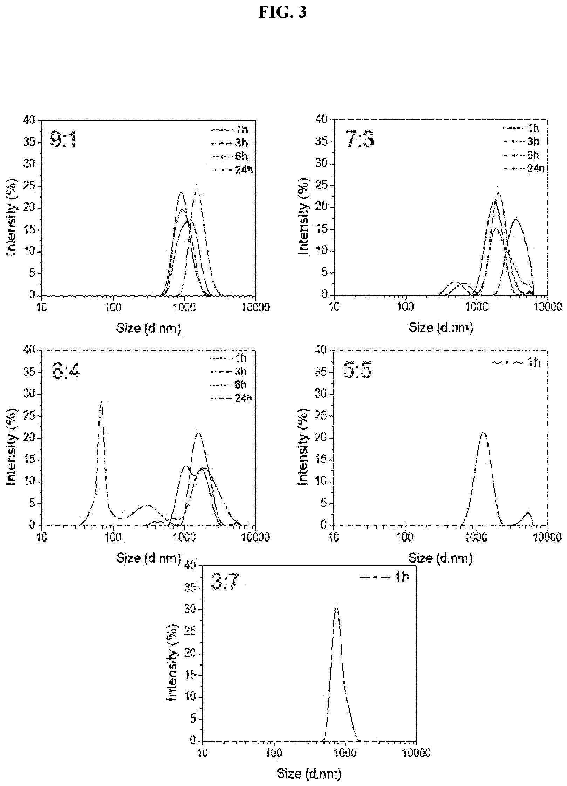

In addition, as shown in FIG. 3, as a result of confirming the size distribution of the microbubbles prepared in the five different ratios, in the cases of the 3:7 and 5:5 ratios, except for the peak at 1h, the size distribution was not shown in the zeta-size due to low stability, and in the case of the 6:4 ratio, it was shown that the microbubbles are instable at 24h. On the other hand, it can be confirmed that, in the case of the 7:3 and 9:1 ratios, the size distribution of the microbubbles were stable, and particularly, in the 9:1 ratio, the microbubbles had the highest stability.

Accordingly, in the present invention, microbubbles were prepared with the DSPC:DSPE-PEG2K-NHS ratio of 9:1, and used for an experiment. Here, the microbubbles were prepared by the method described in Example 2-1, and the concentration of a phospholipid film was 0.5 mg/ml.

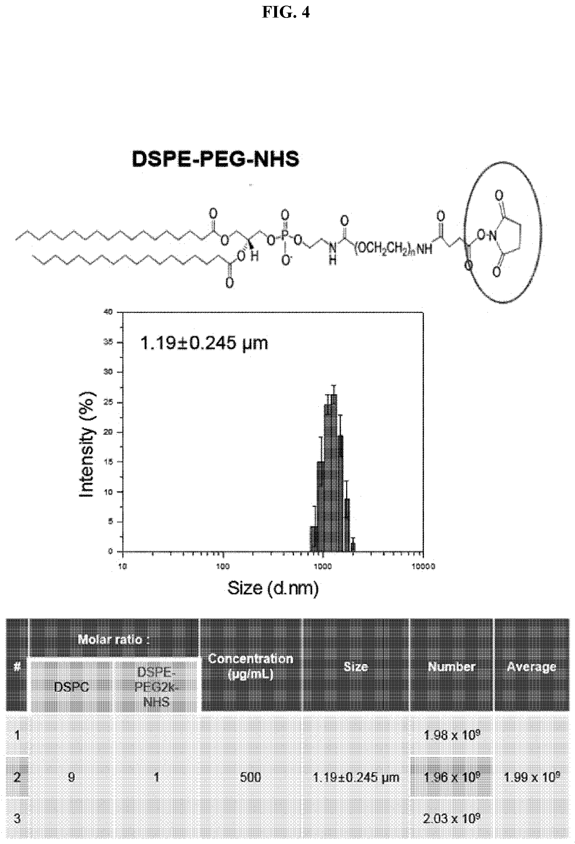

The structure of DSPE-PEG-NHS used in the preparation of microbubbles and the size distribution of microbubbles prepared with the DSPC:DSPE-PEG2K-NHS ratio of 9:1 are shown in FIG. 4, and the size of the microbubbles was 1.19±0.245 µm, and the number of the microbubbles was 1.99 × 10⁹ on average.

### 2-3. Confirmation of stability of microbubbles according to time and temperature

To test the stability of microbubbles (MBs) according to time and a temperature, the numbers of MBs prepared after filling with air (MB with air) and MBs prepared after filling with SF₆ gas (MB with SF₆) were monitored over time under an optical microscope at 4 °C and 25 °C.

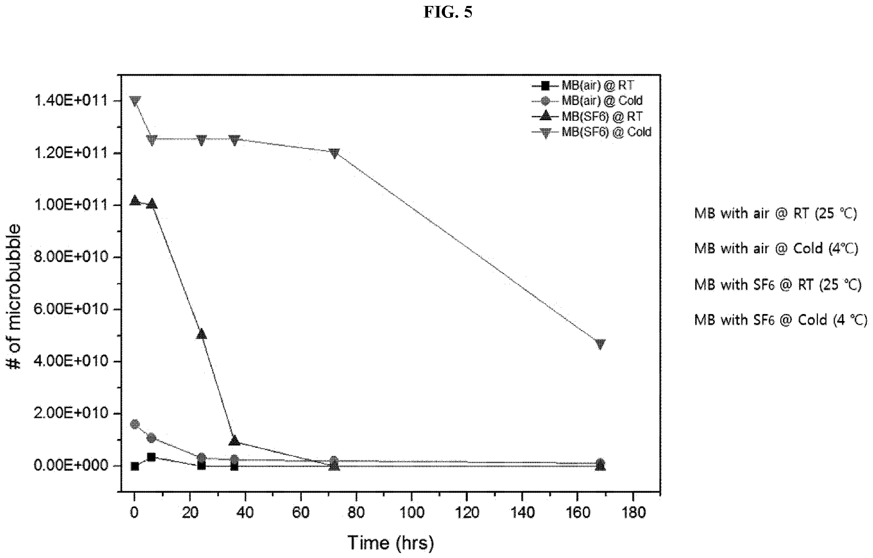

As a result, as shown in FIG. 5, it was confirmed that, in the case of MB with SF₆ (4 °C), the largest number of microbubbles was observed, and the microbubbles were highly stable.

### Example 3. Preparation of immune-microbubble complex

### 3-1. Conditions for microbubble-antibody particles

An immune-microbubble complex (IMC) was prepared by conjugating an anti-PD-L1 antibody to N-hydroxysuccinimide (NHS) of the microbubbles prepared in Example 2-1. Here, on the assumption that the anti-PD-Ll antibody reacts with NHS at 1:1, conditions of an anti-PD-Ll antibody for preparing IMC were confirmed.

In this case, as shown in Table 1 the number of NHS molecules is approximately 100 times larger than that of the PD-L1 antibodies, so, it was expected that antibodies binding to MB would be sufficient when a single dose of the anti-PD-L1 antibodies was 100 to 200 µg.

**[Table 1]**

| **IgG (da)** | **IgG (g/mol)** | **amount of ab used (ug)** | | **amount of ab used (g)** | | **mol** | **mol -> number** |
|---|---|---|---|---|---|---|---|
| 150000 | 150000 | 200 | | 0.0002 | | 1.33333E-09 | 8.02933E+ 14 |
| | | | | | | | |

| **NHS (g/mol)** | **amount of ab used (ug)** | | **amount of ab used (g)** | | **mol** | **mol -> number** | |
|---|---|---|---|---|---|---|---|
| 2890.486 | 290 | | 0.00029 | | 1.00329E-07 | 6.04182E+16 | |

### 3-2. Preparation of immune-microbubble complex

After purification of the microbubbles prepared in Example 2-1, 200 µg of anti-PD-L1 antibodies (herein after, PD-L1 Abs; BioXcell, USA) were conjugated to 1.5 × 10⁹ microbubbles. To remove unconjugated PD-L1 Abs, the purification of the microbubble-antibody conjugate was performed by gradient centrifugation for 5 minutes at 300 rpm, and the size and surface charge were measured by dynamic light scanning (Malvern Zetasizer Nano series, England).

### 3-3. Measurement of binding efficiency of anti-PD-Ll antibody

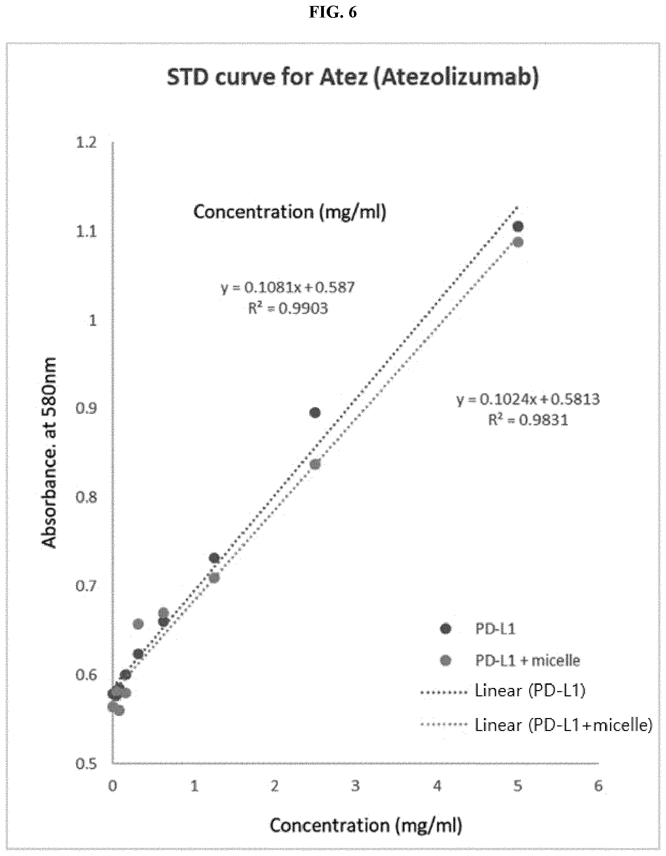

After IMC synthesis, concerning possible errors in the binding strength measurement because of liposome+antibody (Ab) remaining in a filtrate, concentrations of (1) lipid(micelle)+Ab and (2) Ab were first measured. As a result, as shown in FIG. 6, it was confirmed that there was almost no difference between the two groups, and the antibody binding efficiency was confirmed by measuring an unbound antibody present in the filtrate after IMC synthesis by the Bradford assay method.

Specifically, after sufficiently waiting to avoid mixing the antibodies as much as possible in the filtrate, the filtrate was separated through a syringe, mixed with the Bradford solution and left for 5 minutes, followed by measuring the antibodies.

As a result, in the STD curve, there was no difference from the control group (no Ab), and as shown in Table 2 below, the antibody binding efficiency was 99.9% through the Bradford assay, and there was no different between the filtrate and the control, showing that 200 µg of the antibodies were 100% conjugated.

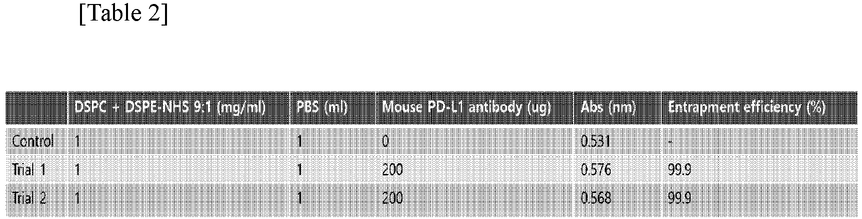

In addition, as a result of measuring the Bradford assay using conjugation with thiol, the antibodies were measured at 0.581 nm and 0.576 nm as shown in Table 3 below.

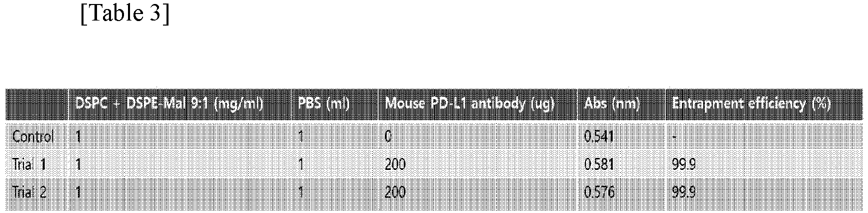

### Example 4. Confirmation of confocal images of immune-microbubble complex (IMC)

### 4-1. Confirmation of confocal images according to concentration of antibody

To confirm the conjugation of an antibody to microbubbles, confocal images of IMC were confirmed.

Specifically, IMC that is prepared by conjugating 10 µL (0.01 mg/ml) of MB by adding 20 to 160 µg of primary antibodies (mouse PD-L1 antibodies) was treated with FITC-tagged secondary antibodies (antibodies binding to the Fc region of the mouse PD-L1 antibody) in an amount that was twice as much as the primary antibodies at 4 °C for 60 minutes. The IMC solution to which the secondary antibodies are conjugated was purified by centrifugation at 3,000 rpm for 5 minutes, followed by dropping the purified product on a slide glass. Then, the IMC was observed with a confocal laser scanning microscope (CLSM).

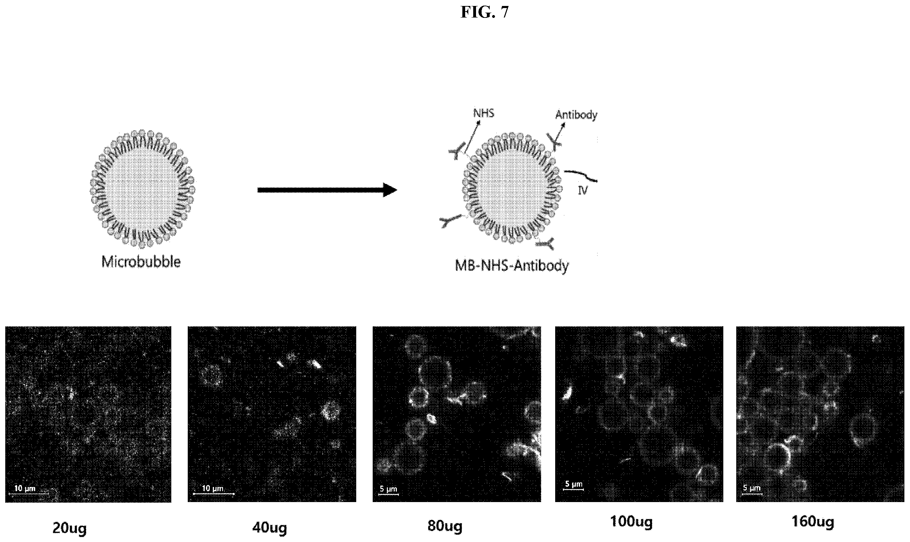

As a result, as shown in FIG. 7, as the concentration of the antibodies increases, fluorescence became more distinct, and it was observed that the anti-PD-Ll antibodies have a high binding strength to MB

### 4-2. Comparison of GFP and DIC images

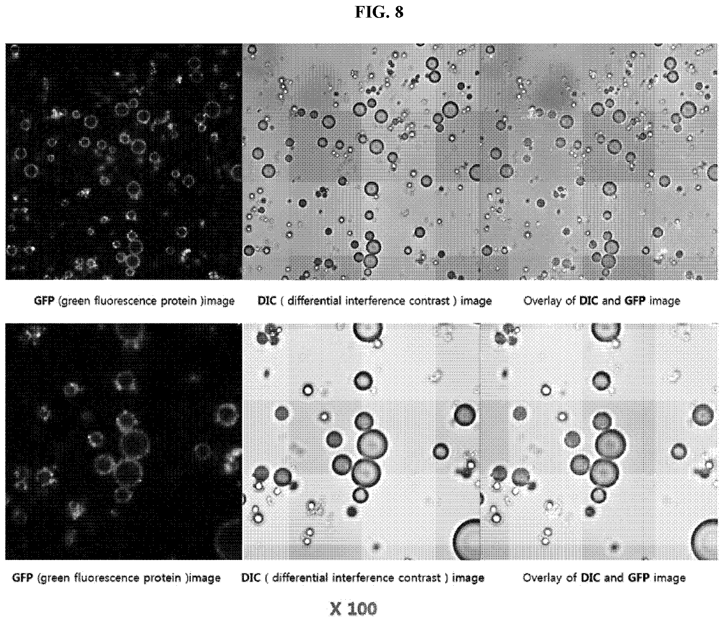

IMC prepared by conjugating 1 mg of MB by adding 1 mg of primary antibodies (anti-PD-Ll antibodies) was treated with 1 mg of GFP-tagged secondary antibodies, and then confocal images thereof were observed by the method described in Example 4-1. Images observed using a differential interference contrast (DIC) microscope and images in which GFP and DIC are combined are shown in FIG. 8.

As shown in FIG. 8, by observing fluorescence on the surface of the microbubbles, it was confirmed that, in the IMC according to the present invention, the antibodies are conjugated to the surface of the microbubbles.

### Example 5. Effect caused by IMC and HIFU

### 5-1. Confirmation of PD-L1 expression in B16F10 cells

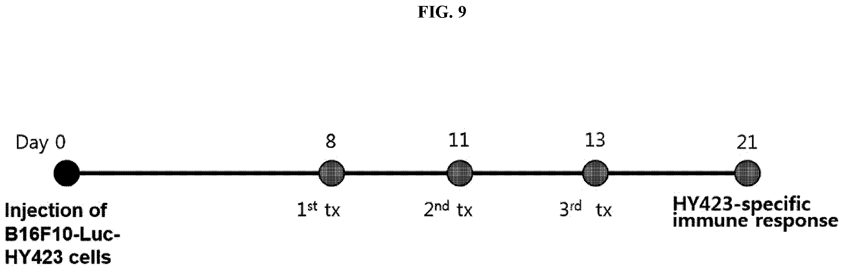

As shown in FIG. 9, an experiment was designed, PD-L1 expression in B16F10 cells was confirmed.

Specifically, B16F10 cells were dissolved in wells, and to detect the expressed PD-L1, 50 µg of anti-PD-Ll antibodies were added. Subsequently, after washing twice with PBS or media, and for confirmation with a confocal microscope, 50 to 100 µg of fluorescence-tagged secondary PD-L1 antibodies were added and left for approximately 2 hours. Afterward, washing was performed again 2 or 3 times, and confocal imaging was performed.

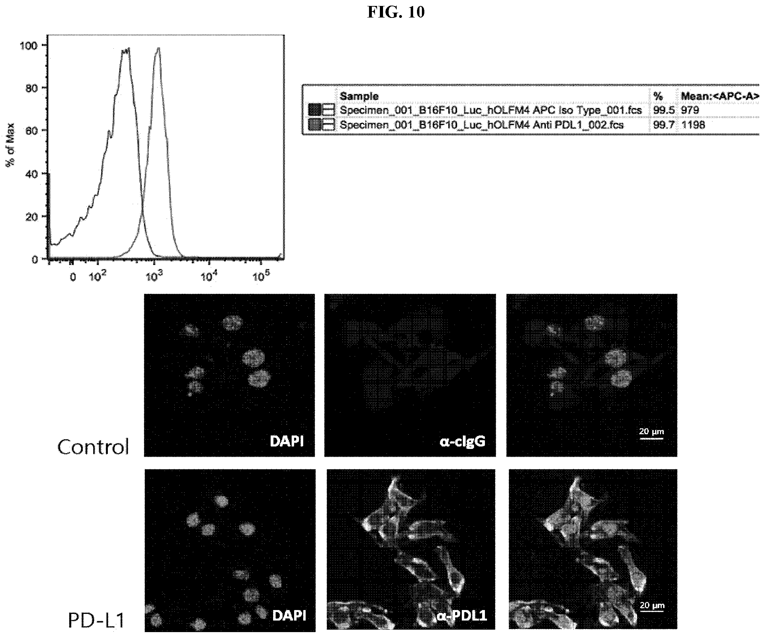

As a result, as shown in FIG. 10, as a result of confirming the expression level ofPD-L1, through fluorescent PD-L1, it was confirmed that PD-L1 is highly expressed on the cell surface by observing PD-L1 stained in red.

### 5-2. Anti-tumor activity by IMC and HIFU

To confirm anti-tumor activity of the IMC prepared in Example 3-2 or when the IMC was treated with HIFU, a CT26wt tumor-harboring BALB/c mouse model established by subcutaneously injecting a CT26wt cell suspension (1 × 10⁶ cells per mouse) into the right flank of 4-week-old BALB/c female mice was used. After the tumor volume reached 50 mm³, the mice were randomized for treatment. Experimental groups were defined as follows:
G1: Isotype Ab only (0.3 mg)
G2: PD-L1 Ab only (0.3 mg)
G3: PBS
G4: HIFU only
G5: Isotype Ab + MB (0.3 mg)
G6: Isotype Ab + MB + HIFU (0.3 mg)
G7: PD-L1 + MB (0.3 mg)
G8: IMC + HIFU (0.3 mg)

As isotype Abs, homogeneous IgG2 Abs without the function of PD-L1 Abs were used as a control.

A tumor size was measured with digital calipers, and tumor tissue was excised for histological analysis at the end of the experiment.

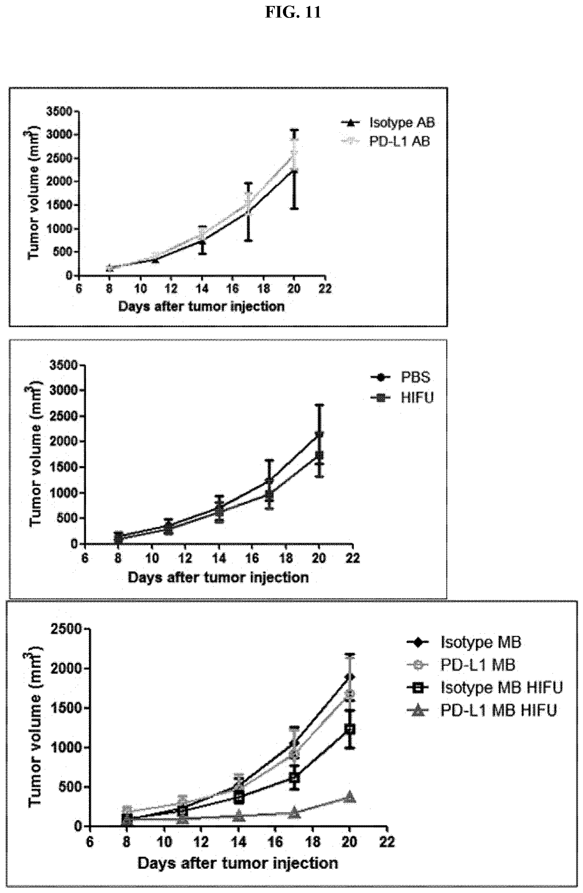

As a result, as shown in FIG. 11, compared to G2, the tumor size of G1 was slightly smaller, but has no significant difference (the top view of FIG. 11), compared to G3, the tumor size of G4 was slightly smaller (the middle view of FIG. 11), and when both of the Isotype Ab + MB group (G5) and the PD-L1 Ab + MB group (G7) were treated with HIFU, the tumor sizes remarkably decreased, and particularly, in the G8 group in which PD-L1 Ab + MB (IMC) was treated with HIFU, the largest decrease in tumor size was shown (the bottom view of FIG. 11).

Therefore, from the above results, it was found that HIFU treatment on the immune-microbubble complex (IMC) prepared in the present invention results in an excellent anti-tumor effect.

### 5-3. Activity for IFN-γ and cytolytic T cells

The IFN-γ assay was performed by extracting plasma or ascites from a mouse and assessing them using a Quantikine mouse IFN-γ assay kit (R&D Systems, Minneapolis, MN) through ELISA.

In addition, for cytolytic T cells, after resuspending cancer cells with PBS, 10 µM carboxyfluorescein succinimidyl ester (CFSE) was added to the resulting suspension to react with the cells at 37 °C for 10 minutes, and an equal amount of FCS at room temperature for 2 minutes was treated to stop the reaction. Subsequently, after washing with AIM-V (+5% human A/B serum) twice, the resulting cells were resuspended with AIM-V+IL2, IL-7 and IL-15 (+5% human A/B serum), and incubated with a 1:10 mixture of dendritic cells (DCs) and cytotoxic T cells (CTL) at 37 °C for 6 hours. Afterward, 1 µg/ml propidium iodide was added, and 10 µl of calibrate beads were added before measurement.

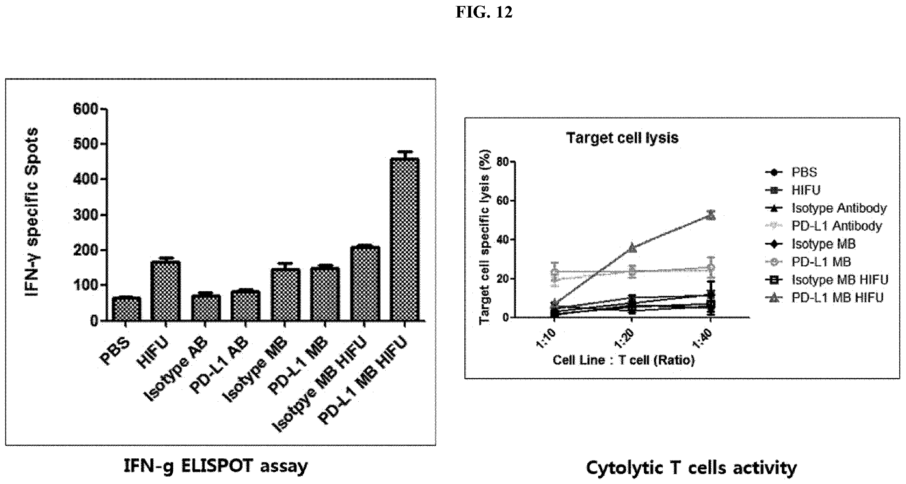

As a result, as shown in FIG. 12, like the result of Example 5-2, it was confirmed that the G8 group in which PD-L1 Ab + MB (IMC) was treated with HIFU shows the highest IFN-γ specific spot, and the highest activity of cytolytic T cells.

Consequently, it can be confirmed that the treatment of the immune-microbubble complex according to the present invention with HIFU results in an immune-enhancing effect.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

An immune-microbubble complex according to the present invention may increase the efficiency of delivering a conjugated antibody and be used in both diagnosis and treatment of cancer, and is expected to be used in the field of immunotherapy, including a contrast effect, half-life improvement, improved drug delivery, a lymphocyte concentration effect, cancer immunotherapy and induction of immunotherapy using ultrasound.

## Claims

1. An immune-microbubble complex, comprising:
microbubbles; and
an immune checkpoint inhibitory antibody conjugated on the surface of the microbubbles, the immune-microbubble complex activating T cells by suppressing immune evasion of tumor cells in an environment in which an immune response is boosted by ultrasound sonophoresis.

2. The complex of claim 1, wherein the conjugation of an antibody with the microbubbles is an amide bond, a bond between thiols, or a biotin-avidin bond.

3. The complex of claim 1, wherein the microbubbles comprise 1,2-distearoylsn-glycero-3-phosphorylcholine (DSPC) and 1,2-distearoyl-sn-glycero-3-phosphoethanolamine (DSPE).

4. The complex of claim 3, wherein the DSPE is 1,2-distearoyl-sn-glycero-3-phosphoethanolamine-N-[succinyl(polyethylene glycol)-2000] (DSPE-PEG2000-NHS).

5. The complex of claim 3, wherein the molar ratio of the DSPC and DSPE is 6 to 9:1 to 4 (mol).

6. The complex of claim 1, wherein the ultrasound is high intensity focused ultrasound (HIFU).

7. The complex of claim 1, wherein the immune checkpoint inhibitory antibody is one or more selected from the group consisting of an anti-programmed death ligand-1 (PD-L1) antibody, an anti-B7-1 antibody, and an anti-B7-2 antibody.

8. A method of preparing an immune-microbubble complex, comprising:
(a) mixing phospholipids with an organic solvent and then hydrating the resulting mixture;
(b) preparing microbubbles by dispersing and stirring the hydrated liposome precursor obtained in step (a); and
(c) conjugating an immune checkpoint inhibitory antibody to the microbubbles.

9. The method of claim 8, wherein the phospholipid comprises 1,2-distearoyl-snglycero-3-phosphorylcholine (DSPC) and 1,2-distearoyl-sn-glycero-3-phosphoethanolamine (DSPE).

10. The method of claim 9, wherein the molar ratio of DSPC and DSPE is 6 to 9:1 to 4 (mol).

11. A drug delivery carrier comprising the immune-microbubble complex of any one of claims 1 to 7.

12. A contrast agent composition for cancer cell-specific ultrasound, magnetic resonance imaging or fluorescence analysis, comprising the immune-microbubble complex of any one of claims 1 to 7.

13. A pharmaceutical composition for preventing or treating cancer, comprising the immune-microbubble complex of any one of claims 1 to 7.

14. The pharmaceutical composition for preventing or treating cancer of claim 13, wherein the immune-microbubble complex is used in combination with ultrasound treatment.

15. The pharmaceutical composition for preventing or treating cancer of claim 14, wherein the ultrasound is high intensity focused ultrasound (HIFU).

16. The pharmaceutical composition for preventing or treating cancer of claim 13, wherein the immune-microbubble complex is cavitated by ultrasound to concentrate lymphocytes near cancer cells.

17. A method of providing information on cancer diagnosis, comprising:
treating a biological sample with the immune-microbubble complex of any one of claims 1 to 7; and
performing ultrasound treatment.

18. The method of claim 17, wherein the ultrasound is high intensity focused ultrasound (HIFU).

19. A method of preventing or treating cancer, comprising:
administering a composition comprising the immune-microbubble complex of any one of claims 1 to 7 to a subject in need; and performing ultrasound treatment.

20. A use of a composition comprising the immune-microbubble complex of any one of claims 1 to 7 for preventing or treating cancer.

21. A use of the immune-microbubble complex of any one of claims 1 to 7 for producing a drug used in cancer treatment.
